(19) Europäisches Patentamt / European Patent Office / Office européen des brevets

(11) **EP 3 661 594 B1**

(12) **EUROPÄISCHE PATENTSCHRIFT**

(45) Veröffentlichungstag und Bekanntmachung des Hinweises auf die Patenterteilung:
**22.10.2025 Patentblatt 2025/43**

(21) Anmeldenummer: **18738249.4**

(22) Anmeldetag: **29.06.2018**

(51) Internationale Patentklassifikation (IPC):
**A61N 1/40** *(2006.01)* **H01P 7/06** *(2006.01)*

(52) Gemeinsame Patentklassifikation (CPC):
**H01P 7/06; A61N 1/40**

(86) Internationale Anmeldenummer:
**PCT/EP2018/067702**

(87) Internationale Veröffentlichungsnummer:
**WO 2019/025095 (07.02.2019 Gazette 2019/06)**

(54) **BIORESONANZFREQUENZ-SIGNALRESONATOR**

BIORESONANCE FREQUENCY SIGNAL RESONATOR

RÉSONATEUR DE SIGNAL POUR FRÉQUENCES DE BIORÉSONANCE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **31.07.2017 DE 102017007280**

(43) Veröffentlichungstag der Anmeldung:
**10.06.2020 Patentblatt 2020/24**

(73) Patentinhaber: **Apere GmbH & Co. KG 24235 Wendtorf (DE)**

(72) Erfinder:
• **KEYMER, Martin**
  **24235 Wendtorf (DE)**
• **KREISL, Peter**
  **82396 Pähl (DE)**
• **MEDINGER, Walter**
  **3500 Krems (AT)**

(74) Vertreter: **Pfenning, Meinig & Partner mbB Patent- und Rechtsanwälte Joachimsthaler Straße 10-12 10719 Berlin (DE)**

(56) Entgegenhaltungen:
EP-A2- 0 436 840        EP-A2- 0 885 628
DE-A1- 102015 006 368   DE-U1- 202013 100 991
US-A- 2 460 090         US-A- 3 161 840
US-A1- 2011 298 566

• HARTL HELMUT: "Medizinisch verwertbare Frequenzen aus Pflanzen. Ein Forschungs-Vorbericht", CARINTHIA II, vol. 196, 1 January 2006 (2006-01-01), pages 75 - 84, XP055975180
• MAUERMANN JUTTA: "Wie Frequenzen helfen können. Erfolge der Frequenztherapie und begleitende Empfehlungen aus der Naturheilkunde", 1 January 2014 (2014-01-01), pages 1 - 108, XP055975184, ISBN: 978-1-907469-18-3, Retrieved from the Internet <URL:https://www.biocenter24.com/mediafiles/Ebooks/Downloads/Info4Life-wie_frequenzen_helfen_koennen.pdf> [retrieved on 20221026]

# EP 3 661 594 B1

**Beschreibung**

Hintergrund der Erfindung

Nach herkömmlicher Ansicht wirkt eine Bioresonanzbehandlung dadurch auf den menschlichen Körper, dass ein körpereigenes Bioresonanzfrequenz-Signal gleichphasig oder invertiert oder beides im zeitlichen Wechsel oder aufgespalten in verschiedene Phasenanteile (unter Invertierung der unharmonischen Anteile) dem Patienten wieder zugeführt wird.

Die Resonanz zählt als grundlegender Mechanismus des Energieaustausches zwischen oszillierenden Systemen zum wohlbekannten Kenntnisstand der klassischen Physik. Ein besonderes Kennzeichen der Resonanz besteht darin, dass sich kleine Energiebeträge kumulieren und schließlich zu drastischen Ergebnissen führen können (Resonanzkatastrophe).

Die Voraussetzung dafür, dass ein Oszillator in einem anderen schwingungsfähigen System (dem Resonator) Resonanz (d.h. ein Mit-Schwingen) hervorruft, besteht darin, dass die Frequenz des Oszillators mit einer Eigenfrequenz des Resonators übereinstimmt.

Die Atom- und Molekülspektroskopie hat sehr viel über die Schwingungen der mikroskopischen Oszillatoren (Atome, Moleküle) enthüllt, sowie die darin steckende Information (z.B. über Molekülstrukturen, Rotationen und Vibrationen von Molekülen usw.). Das Schwingungsspektrum des Menschen besteht aus den Schwingungen dieser elementaren Bestandteile sowie der komplexeren biologischen Strukturen (Makromoleküle, Cluster, Zellorganellen, Zellen, Organe usw.) und des gesamten Körpers, auf dessen Eigenschwingungsmoden die Meridianstruktur zurückzuführen ist. Die Gesamtheit dieser Oszillationen bildet das Biofeld des Menschen (Bioplasma).

Die Resonanz zwischen Oszillatoren ist ein Mechanismus der Signalübertragung ohne strukturelle Änderungen oder Massetransport. Damit ein Resonator in Resonanz gehen kann, ist eine gewisse Mindestamplitude der Schwingung des anregenden Oszillators erforderlich. Die Qualität der Übertragung hängt aber von der Schärfe des Signals ab. Die schärfsten elektromagnetischen Signale erhält man dadurch, dass man einem schwingungsfähigen System Randbedingungen auferlegt, die nur ganz bestimmte Schwingungsfrequenzen zulassen. Dies erreicht man im einfachsten Fall durch Ausbildung stehender elektromagnetischer Wellen, im komplexeren dreidimensionalen Fall durch Hohlraumresonanzen. Die Wirkung eines Hohlraumresonators ist mit derjenigen eines Resonanzkörpers bei einem Musikinstrument zu vergleichen, der ein bestimmtes Schwingungsspektrum erzeugt und dadurch dem Instrument seine spezifische Klangfarbe verleiht. Die Besonderheit von stehenden Wellen und Hohlraumresonanzen liegt in der festen Phasenkopplung der einzelnen Oszillatoren, die man als Kohärenz bezeichnet.

Der Erfolg der Bioresonanz - und zwar bereits der konventionellen -, beruht auf der Kohärenz der Signale, die ursprünglich vom Patienten selbst oder von getesteten Substanzen abgegriffen wurden, und auf der Wechselwirkung im Interferenzfeld zwischen dem Eingangssignal, dem Ausgangssignal und dem Biofeld des Patienten (Bioplasma).

Hohlraumschwingungsmoden elektromagnetischer Felder haben in der Biologie überragende Bedeutung. So konnte von Popp und Rattemeyer gezeigt werden, dass die vor einer Zellteilung (Mitose) auftretende "mitotische Spindel", eine altbekannte, aber lange unerklärte Anordnung der Chromosomen wie an "Strippen", einer elektrischen Infrarot-Transversalschwingungsmode in der Zelle entspricht. Der Körper und seine Strukturen (z.B. Zellen) zeigen selbst das Verhalten von Hohlraumoszillatoren mit kohärenten Eigenschwingungsmoden.

Erkenntnisse wie diese belegen, dass kohärente durch Hohlraumschwingungen erzeugte Signale mit Eigenschwingungen des Körpers in Resonanz und damit in eine "erweiterte" Kohärenz gehen können.

Bei der herkömmlichen apparativen Ausführung der Bioresonanztherapie treten 1. Störungen der Kohärenz des Patientensignals und des therapeutischen Signals durch unerwünschte äußerliche Einwirkung von EMF (elektrischen, magnetischen und elektromagnetischen Feldern) ein, und 2. wird die Interferenz kohärenter Signale (nämlich der vom Patienten und aus anderen Signalquellen wie Therapieampullen oder technischen Oszillatoren abgeleiteten elektromagnetischen Signale einerseits und des Biofeldes des Patienten andererseits) eher zufällig als gezielt herbeigeführt.

Früher versuchte Lösungen des 1. Problems durch die Verwendung geschirmter Kabel haben dazu geführt, dass die nach dem 2. Punkt wesentliche Interferenz verhindert wurde und ein verminderter therapeutischer Erfolg eintrat. Deshalb ist heute in der Bioresonanztherapie die Verwendung ungeschirmter Kabel außerhalb eines Bioresonanzgerätes obligat, wodurch aber das 1. Problem ungelöst bleibt. Weiters ist bei der herkömmlichen Einführung der Signale von Therapieampullen oder technischen Oszillatoren in den Signalweg der Bioresonanztherapie keine Synchronisation oder gar Kohärenz mit dem Patientensignal verwirklicht.

Bringt man ein Patientensignal und/oder Signal einer anderen Vorlage (z.B. getestete Ampulle) und/oder ein zusätzliches technisch generiertes Signal in einen elektromagnetisch resonanzfähigen Raum, kann dieser als Bioresonanzfrequenz-Signalresonator bezeichnete Raum in seinen Dimensionen (zumindest der Länge nach) verstellbar sein und selektiert auf diese Art unterschiedliche Frequenzen als Eigenschwingungsmoden, wobei eine kohärente elektromagnetische Oszillation und eine maximale Signalschärfe erzielt werden. Der durch die Auswahl von Eigenschwingungen gegebene Filterungseffekt kann in vorteilhafter Weise genützt werden, um das ableitbare Signal auf bestimmte Körperresonanzen (z.B. von Organen oder Meridianen) abzustimmen.

Die wichtigsten Möglichkeiten, kohärente elektromagnetische Signale zu erzeugen, sind Hohlraumresonanzen und stehende Wellen. Im Resonator werden beide Prinzipien in Form von Eigenschwingungen eines zylindrischen (gegebenenfalls auch in seiner Form veränderbaren) Resonators verwirklicht, wobei der Resonator mit den als Komponenten des therapeutischen Signals bestimmten Eingangssignalen gespeist wird und der Eintritt der Resonanz durch einen vektoriellen Netzwerkanalysator (VNA) angezeigt werden kann.

Es genügt für die Erzeugung einer stehenden Welle, die Strahlung eines Hochfrequenz- (HF-) Senders senkrecht auf eine Metallwand treffen zu lassen. Durch Reflexion an dieser Wand bildet sich eine rücklaufende Welle, die sich mit der ursprünglichen (hinlaufenden) überlagert. Im Raum vor der Wand ist die resultierende stehende Welle mit einem Dipolempfänger hinsichtlich der elektrischen Komponente, mit einer Induktionsschleife hinsichtlich der magnetischen Komponente gut nachweisbar.

Die Grundmode der stehenden Welle ist durch einen einzigen Schwingungsbauch in der Mitte des Resonanzraumes und je einen Schwingungsknoten an den Enden gekennzeichnet.

Die physikalische Lehre besagt, dass es durch die Metallwand (ideal wäre eine spiegelnde Oberfläche) zu einer "Reflexion am festen Ende" mit einem Phasensprung um 180° kommt. Eigenmoden stehender Wellen haben einen Abstand von einem Schwingungsknoten zum nächsten (und von einem Schwingungsbauch zum nächsten), der einem ganzzahligen Vielfachen der halben Wellenlänge $\lambda$ der ursprünglichen Welle entspricht:

$$h = p\,\frac{\lambda}{2}$$

$h$ kann in diesem Fall als die Länge eines zylindrisch gedachten Resonators (Resonanzraumes) aufgefasst werden, das ist der Abstand zwischen den beiden reflektierenden Flächen des Resonators. In einem zylindrischen Resonanzraum wäre $h$ also als Zylinderhöhe zu betrachten. $p$ ist eine natürliche Zahl. Ist $p$ ungerade, so befindet sich ein Schwingungsbauch in der Mitte des Resonanzraumes.

Drückt man die Wellenlänge $\lambda$ durch die Lichtgeschwindigkeit $c$ und die Frequenz $f$ aus, so erhält man nach Umformen folgende Beziehung für die Eigenfrequenz der $p$-ten Schwingungsmode:

$$f_p = \frac{cp}{2h}$$

Sind wie beim Resonator in einem zylindrischen Hohlleiter senkrecht zur Achse zwei verstellbare metallische Reflektoren eingebaut und wird nun die Zylinderhöhe h zwischen diesen beiden Stirnflächen variiert, so tritt unter Einbeziehung der zusätzlichen Reflexionen an der metallischen Zylinderwand genau dann der Resonanzfall mit Ausbildung einer kohärenten transversalen magnetischen oder elektrischen Hohlraum-Schwingungsmode TM bzw. TE einer an den Eingang gelegten Schwingung ein, wenn eine der folgenden Beziehungen erfüllt ist:

$$f_{mnp}^{\mathrm{TM}} = \frac{c}{2\pi}\sqrt{\left(\frac{j_{m,n}}{R}\right)^2 + \left(\frac{p\pi}{h}\right)^2}$$

$$f_{mnp}^{\mathrm{TE}} = \frac{c}{2\pi}\sqrt{\left(\frac{j'_{m,n}}{R}\right)^2 + \left(\frac{p\pi}{h}\right)^2}$$

$f^{TM}$ bzw. $f^{TE}$ ist die jeweilige Frequenz, die eine der obigen Beziehungen bei gegebener Zylinderhöhe $h$ und gegebenem Zylinderradius $R$ sowie passenden natürlichen Zahlen $m$, $n$, $p$ erfüllt. $j_{m,n}$ bzw. $j'_{m,n}$ sind die $n$-ten Nullstellen der $m$-ten Bessel'schen Funktion $J_m$ bzw. ihrer ersten Ableitung $J'_m$.

Wird nun ein komplexes Signal in den Hohlraum eingespeist (dies kann auch durch Einspeisung mehrerer Signale über mehrere Eingänge erfolgen), so werden aus diesem Signal bzw. diesen Signalen jene Frequenzen ausgefiltert, die nach den angegebenen Formeln mit dem Zylinderradius $R$ und der Zylinderhöhe h kompatibel sind.

Stand der Technik

Aus der DE 27 57 716 A1 ist ein Hohlraumresonator für Mikrowellensysteme mit einem zylindrischen Hohlraum, der bei einer ersten Mode in Resonanz ist, bekannt, wobei der Hohlraum verschiedene Mittel enthält, die eine entsprechende Gestaltung bereitstellen, sodass eine zweite Mode in Resonanz damit gelangt. Durch verschieben der Mittel können zwar verschiedene einzelne Frequenzen gefiltert werden, aufgrund seiner baulichen Kompaktheit ist dieser Hohlaumresonator aber nicht als Bandfilter mit automatischem Frequenzdurchlauf einzelner, selektierbarer Resonanzfrequenzen geeignet.

Die DE 40 20 881 A1 offenbart einen Resonanzschwinger mit einem aktiven scheibenförmigen Schwingerteil, der unter Bildung eines Koppelschwingers mit einem passiven Schwingerteil in Berührung steht, wobei die Resonanzfrequenz hauptsächlich von einer Flüssigkeits-Festkörpersäule zwischen den Schwingerteilen bestimmt wird, deren Länge kontinuierlich veränderbar ist, sodass sich kontinuierliche Veränderungen der Resonanzfrequenzen erzielen lassen. Aufgrund der Notwendigkeit, der veränderbaren Flüssigkeitssäule benötigt der Resonanzschwinger zusätzliche hydraulische Elemente, z. B. Ausgleichsbehälter, Kolben, Dichtungen etc. Der Anwendungsbereich dieses Resonanzschwingers liegt hauptsächlich im Ultraschallbereich, da eine Flüssigkeit mit möglichst großer Dichte benötigt wird.

Die Gebrauchsmusterschrift DE 20 2013 100 991 U1 ist mit einer Therapievorrichtung zur Erzeugung elektromagnetischer Schwingungen befasst. Die deutsche Offenlegungsschrift DE 10 2015 006 368 A1 betrifft ein Bandpassfilter mit Hohlraumrersonator. U.S. Patent 3,161,840 beschreibt Resonatoren mit abstimmbarer Kavität gemäß der Präambel des Anspruchs 1.

Aus der US 2 460 090 A ist ein abstimmbarer Hohlraumresonator bekannt, mit dem verschiedene Resonanzfrequenzen einstellbar sind. Dazu weist dieser einen axial verstellbaren, konisch zulaufenden zylindrischen Mantel auf, in den eine ortsfeste Abdeckplatte und eine ebenfalls axial verstellbare Abdeckplatte hineinragen, um den Resonanzraum zu begrenzen. In der ortsfesten Abdeckplatte sind zwei Schleifen zur Energieübertragung so angeordnet, dass sie symmetrisch zur Mitte der Abdeckplatte und zum zylindrischen Mantel liegen. Zur Justierung einzelner Resonanzfrequenzen können der zylindrische Mantel und die nicht ortsfeste Abdeckplatte jeweils über einzelne Ritzel und mittels vorgegebener Skalen händisch verstellt werden. Ein automatischer Durchlauf mit vorgegebenen Einstellungen für bestimmte Resonanzfrequenzen, wie sie insbesondere zur Durchführung von Bioresonanztherapien benötigt werden, ist mit dieser bekannten Konstruktion nicht möglich.

Die US 2 281 550 A beschreibt ein elektrisches Schaltelement, welches bei elektromagnetischen Wellen höchster Frequenzen die Schaltverluste verringern soll. Hieraus ist ein abstimmbarer, zylindrischer Hohlraumresonator entnehmbar. Dabei sind in einem zylindrischen Hohlraum ein geeignetes Gitter und ein verschiebbarer Kolben mit Kolbenstange oder zwei verschiebbare Kolben mit Kolbenstangen vorgesehen, um einen verstellbaren, an die jeweilige Schaltfrequenz angepassten Resonanzraum zu bilden. Weitere bauliche Maßnahmen zur Verstellung der Kolben bzw. Einstellung der Resonanzfrequenzen sind nicht aufgeführt.

Aufgabe und Lösung

Aufgabe der Erfindung ist es, einen Bioresonanzfrequenz-Signalresonator zur Verfügung zu stellen, der den vorstehenden theoretischen Anforderungen genügt und den Schwingungsbauch verschiedener stehender Wellen bzw. Hohlraumschwingungsmoden exakt zu definieren vermag, um eine optimale Auskopplung zu erreichen und einen automatischen Bioresonanzfrequenzumlauf in einfacher Weise zu ermöglichen.

Diese Aufgabe wird mit den Merkmalen des Patentanspruches 1 gelöst. Die Merkmale in den Unteransprüchen enthalten zweckmäßige oder vorteilhafte Ausgestaltungen der Erfindung.

Vorteile der Erfindung

Die Merkmale des Patentanspruches 1 schaffen die Möglichkeit, über Hohlraumresonanzen auch gezielt technisch erzeugte Signale einzubringen, sie in eine kohärente Wechselwirkung mit eingangsseitigen Signalen eines Patienten und aus klassischen BRT-Vorlagen (Patient, Ampullen...) stammende Signale zu bringen und schließlich ausgangsseitig in Form eines kohärenten Signals wieder in Resonanz mit dem Patienten zu bringen.

Die gezielt erzeugten Signale weisen Frequenzen zwischen 0,55 mHz und 730 MHz auf, deren Harmonische mit einem $\lambda/_2$ von 1,39 cm bis 40,43 cm genützt werden.

Durch Variation der Resonanzkörperlänge (1,39 cm bis 40,43 cm) und der ganzen Zahl p werden Resonanzfrequenzen gesucht, die den Frequenzen der Meridiane und Sammelgefäßen oder ihren Harmonischen entsprechen. Einige markante Werte für Frequenzen und den dazugehörigen harmonischen $\lambda/_2$-Werten sind der nachstehenden Tabelle 1 zu entnehmen:

Tabelle 1

| Bezeichnung | Frequenz | | $\lambda/2$ | |
|---|---|---|---|---|
| Magen re. (ELF: li.) | 22,00 | MHz | 40,43 | cm |
| Lymphe | 3,00 | MHz | 39,58 | cm |
| Organ Degen. | 3,90 | MHz | 39,17 | cm |
| Lunge | 24,00 | MHz | 35,99 | cm |
| Gelenks Degen. | 15,00 | MHz | 33,71 | cm |
| Lenkergefäß | 148,00 | MHz | 31,21 | cm |
| Fettige Degen. | 36,00 | MHz | 30,14 | cm |
| Lunge | 24,00 | MHz | 28,71 | cm |
| Blase | 270,00 | MHz | 26,34 | cm |
| Niere | 0,95 | mHz | 23,18 | cm |
| Herz | 384,00 | MHz | 21,54 | cm |
| Leber | 240,00 | MHz | 19,39 | cm |
| Kreislauf | 12,50 | MHz | 19,39 | cm |
| Allergie | 98,00 | MHZ | 16,97 | cm |
| Nerv. Degen. | 0,55 | mHz | 16,28 | cm |
| Haut Degen. | 3,50 | mHz | 14,57 | cm |
| Dienergefäß | 730,00 | MHz | 13,20 | cm |
| Dünndarm | 25,00 | mHz | 3,10 | cm |
| Dickdarm/Milz/Pankreas Frequ. | 55,00 | mHz | 1,39 | cm |

Wie aus der Tabelle ersichtlich, wurde eine Auswahl getroffen, die es erlaubt, in einem Linearmaßstab möglichst gleichmäßig über die Maximallänge von 40,43 cm verteilt Resonanzmaxima (Schwingungsbäuche) für alle angegebenen Bioresonanzfrequenzen, die den Frequenzen der Meridiane und Sammelgefäßen oder ihren Harmonischen entsprechen, abzubilden, was einen automatischen Durchlauf begünstigt.

Das präzise geometrische Justieren der gleichen Abstände vom Mittenabgriff zu den Spiegeln ist durch die Erfindung in einfacher und sicherer Weise gelöst, da nur die Spiegel axial verstellt werden.

Die wenigen dazu benötigten Mittel gibt Patentanspruch 2 an.

Zur einfacheren Handhabung des Bioresonanzfrequenz-Signalresonators dient der Grundrahmen gemäß Patentanspruch 3.

Die Ausbildung der Leitungen zur Signalübertragung nach Patentanspruch 4 schirmt die im Inneren des Bioresonanzfrequenz-Signalgenerators verlaufenden Signalleitungen vor störenden elektromagnetischen Feldern ab.

Die Ausgestaltung des Mittenabgriffes nach Patentanspruch 5 verbessert die Messempfindlichkeit und stellt sicher, dass auch sehr geringe Signale abgreifbar und auskoppelbar sind.

Die Merkmale des Patentanspruches 6 verdeutlichen die klare und einfache Konstruktion.

Die Werkstoffangabe im Patentanspruch 7 gibt einen kostengünstigen Werkstoff an, die Oberflächengestaltung der Spiegel gewährleistet eine optimale Erzeugung der stehenden elektromagnetischen Welle.

Die im Patentanspruch 8 aufgeführten mechanischen Mittel garantieren, dass die Spiegel stets über gleiche Wegstrecken aber in entgegengesetzten Richtungen verschieb- und justierbar sind.

Die vorteilhafte Führung der Koaxialleitungen nach Patentanspruch 9, besonders mit den Leistungsketten, sichert eine einwandfreie Anpassung der Koaxialleitungen an die Bewegungen der Spiegel, sodass ein Verstricken der Koaxialleitungen ausgeschlossen ist.

Die Bedienungseinheit am oder im Grundrahmen nach Patentanspruch 10 vereinfacht die Bedienung des Bioresonanzfrequenz-Signalresonators, sodass weitere Geräte nicht benötigt werden. Mittels der Tastatur können die verschiedenen Betriebsweisen unmittelbar eingegeben werden. Das Display oder der Touchscreen informieren über die eingekoppelten und ausgekoppelten Signale, sodass möglicherweise die Betriebsweise umgehend modifiziert werden kann. Ausführungsbeispiel

Im Folgenden wird eine bevorzugte Ausführungsform der Erfindung unter Bezugnahme auf die Figuren 1 bis 3 näher erläutert.

Dabei zeigen:

**Figur 1:** Ein Ausführungsbeispiel des erfindungsgemäßen Bioresonanzfrequenz-Signalresonators in einer Stellung mit maximalem Spiegelabstand im vertikalen medialen Längsschnitt

**Figur 2:** Ein Ausführungsbeispiel des erfindungsgemäßen Bioresonanzfrequenz-Signalresonators in einer Stellung mit mittlerem Spiegelabstand im vertikalen medialen Längsschnitt

**Figur 3:** Ein Ausführungsbeispiel des erfindungsgemäßen Bioresonanzfrequenz-Signalresonators in einer Stellung mit einem fast minimalem Spiegelabstand im vertikalen medialen Längsschnitt

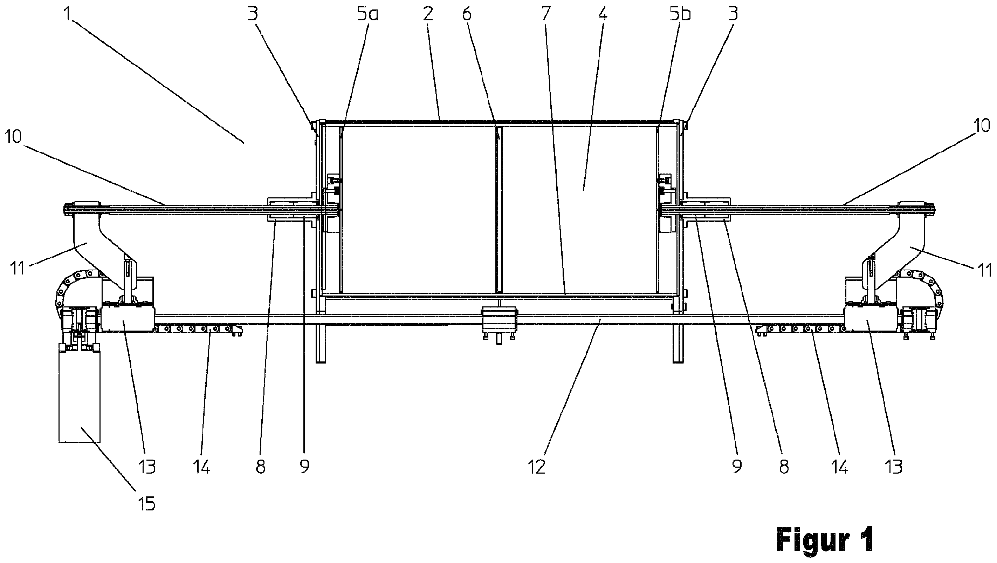

Der in Figur 1 dargestellte Bioresonanzfrequenz-Signalresonator (1) weist einen aus einem magnetisch absorbierenden Metall bestehenden Zylindermantel (2) mit kreisförmigen Abschlussblechen (3) auf, die einen zylindrischen Hohlraum (4) einschließen. Durch den Zylindermantel (2) ist eine Mittellinie festgelegt. Entlang dieser Mittellinie sind innerhalb des Zylindermantels (2) zwei Kreisscheiben, die als Spiegel (5a, 5b) ausgebildet sind, vorhanden. Diese Spiegel (5a, 5b) stehen einander gegenüber.

Weiterhin ist innerhalb des Zylindermantels (2), in der Mitte der Mittellinie, ortsfest ein ringförmigen Mittenabgriff (6) zum Auskoppeln von elektromagnetischen Signalen vorhanden.

Der Mittenabgriff (6) hat die Form eines Ringes und besteht aus Kupfer als Vollmaterial oder kann als kupferne Spule ausgebildet sein. Sein Innendurchmesser liegt bei etwas über 250 mm, damit die Spiegel (5a, 5b) gegebenenfalls in diesen hineingefahren werden können, wenn der geringste Abstand der Spiegel (5a, 5b) angefahren werden muss, wobei diese in den Mittenabgriff (6) hineinfahren.

Die Innenseiten des Zylindermantels (2) und der Abschlussbleche (3) sind mit einer elektromagnetischen Abschirmung (7) beschichtet, die aus dem Werkstoff Kupfer besteht. Diese Abschirmung (7) schützt zwischen den Spiegel (5a, 5b) ausgebildete elektromagnetische stehende Welle vor äußeren, störenden Einflüssen und bildet zugleich eine leitfähige Wand des zylindrischen Hohlraumes.

Auf den Außenseiten der Abschlussbleche (3) befinden sich Führungsflansche (8) mit jeweils einer zentralen Bohrung (9). Mittels dieser Bohrungen (9) sind Hohlwellen (10) axial verschiebbar geführt. Auf den in den Hohlraum (4) ragenden Enden der Hohlwellen (10) sind die Spiegel (5a, 5b) angeordnet. An den äußeren Enden der Hohlwellen (10) sind Träger (11) angebracht.

Unterhalb des Zylindermantels (2) verläuft parallel zu seiner Mittellinie eine handelsübliche Zahnriemenachse (12), die über zwei Schlitten (13) verfügt. Auf diesen ist jeweils ein Träger (11) fest montiert. Weiterhin verfügt die Zahnriemenachse (12) über einen Elektromotor (15), der einen umlaufenden Zahnriemen antreibt. Das Obertrum dieses Zahnriemens ist mit dem einen Schlitten (13), das Untertrum mit dem anderen Schlitten (13) verbunden.

Je nach Laufrichtung des Zahnriemens bewegen sich die Schlitten (13) gleichzeitig über gleiche Wegstrecken aber in entgegengesetzter Richtung. Da auf den Schlitten (13) die Träger (11) montiert sind, überträgt sich die Bewegung der Schlitten (13) über die Träger (11), die Hohlwellen (10) auf die Spiegel (5a) und (5b). Dieses Antriebsgetriebe gewährleistet, dass die Spiegel (5a) und (5b) immer synchron zur Mitte der Mittellinie verschiebbar sind. Weiterhin sichert dies, dass immer der Abstand von Spiegel (5a) zum Mittenabgriff (6) der gleiche ist, wie der Abstand des Spiegels (5b) zum Mittenabgriff (6).

Die Zahnriemenachse (12) ihrerseits ist auf einem nicht dargestellten Grundrahmen fest montiert, der ebenfalls den Zylindermantel (2) trägt.

An den Schlitten (13) sind jeweils Leitungsketten (14) angebracht, deren entgegengesetztes Ende am Grundrahmen befestigt ist.

An den voneinander abgewandten Seiten der Spiegel (5a, 5b) sind jeweils Leitungen zur Signalübertragung angelötet. Diese Leitungen verlaufen durch die Hohlwellen (10), die Leitungsketten (14) und enden in am Grundrahmen vorhandenen zugehörigen Anschlussbuchsen (nicht dargestellt).

Auch ist an dem Mittenabgriff (6) eine Leitung zur Signalübertragung angelötet, die ebenfalls an einer Anschlussbuchse am Grundrahmen endet (nicht dargestellt).

Diese Leitungen können als Koaxialleitungen ausgeführt sein. Die Anschlussbuchsen sind als Koaxialbuchsen ausgeführt. Die zu den Spiegeln (5a und 5b) verlaufenden Leitungen übertragen das einzukoppelnde Signal, die Leitung vom Mittenabgriff (6) überträgt das auszukoppelnde Signal.

Die Spiegel (5a, 5b) haben die Form einer Kreisscheibe und bestehen aus dem kostengünstigen Werkstoff FR4, wie er für Leiterplatten üblicherweise verwendet wird und sind beidseitig mit Kupfer beschichtet. Die einander zugewandten Seiten der Spiegel (5a, 5b) weisen zur besseren elektromagnetischen Wirksamkeit eine aufgedampfte Goldschicht auf. Als geeignet hat sich ein Durchmesser von 250 mm für die Spiegel (5a, 5b) erwiesen.

Eine in den Figuren ebenfalls nicht dargestellte programmierbare Bedienungseinheit ist am Grundrahmen außerhalb des Zylindermantels (2) befestigt und besitzt eine Tastatur, Speicher und ein Display oder Touchscreen. Damit wird der Elektromotor (15) angesteuert. Das Display oder Touchscreen zeigt mindestens den jeweils vorhandenen Spiegelabstand an, die zugehörige, eingekoppelte Bioresonanzfrequenz sowie das vom Mittenabgriff (6) kommende ausgekoppelte Signal an. Es können einzelne vorgebbare Abstände der Spiegel (5a, 5b) ortsfest angefahren werden, entsprechend den ausgewählten Resonanzfrequenzen. Auch ist eine kontinuierliche Verstellung der Spiegelabstände programmierbar, um einen automatischen Resonanzfrequenzdurchlauf ablaufen zu lassen.

Die Oberflächen der Spiegel (5a) und (5b) erscheinen in den Figuren planar sind dies aber nicht. Erfindungsgemäß weisen diese Oberflächen nämlich eine parabolische Form auf.

Bei der Anwendung werden die Abstände der Spiegel (5a, 5b) zueinander bestimmt durch $\lambda/2$ der eingekoppelten Bioresonanzfrequenz oder deren Harmonischen, zum Mittenabgriff (6) durch $\lambda/4$ der Bioresonanzfrequenz oder deren Harmonischen. Dabei übernimmt der eine Spiegel, z.B. (5a), die Funktion einer Sendeantenne, der andere Spiegel (5b) die Funktion eines Spiegels (Reflektors) für die elektromagnetische Welle, sodass eine stehende Welle sich ausgebildet. Dann befindet sich der "Bauch" der ungeraden Moden dieser stehenden Welle bei $\lambda/4$ und das dadurch im Mittenabgriff (6) detektierte maximale Signal ist auskoppelbar.

Figur 1 zeigt den Bioresonanzfrequenz-Signalresonator (1) im vertikalen medialen Längsschnitt im Zustand des weitesten Spiegelabstands von 40,43 cm, dem die Bioresonanzfrequenz des Magens zugeordnet ist (s. Tabelle 1).

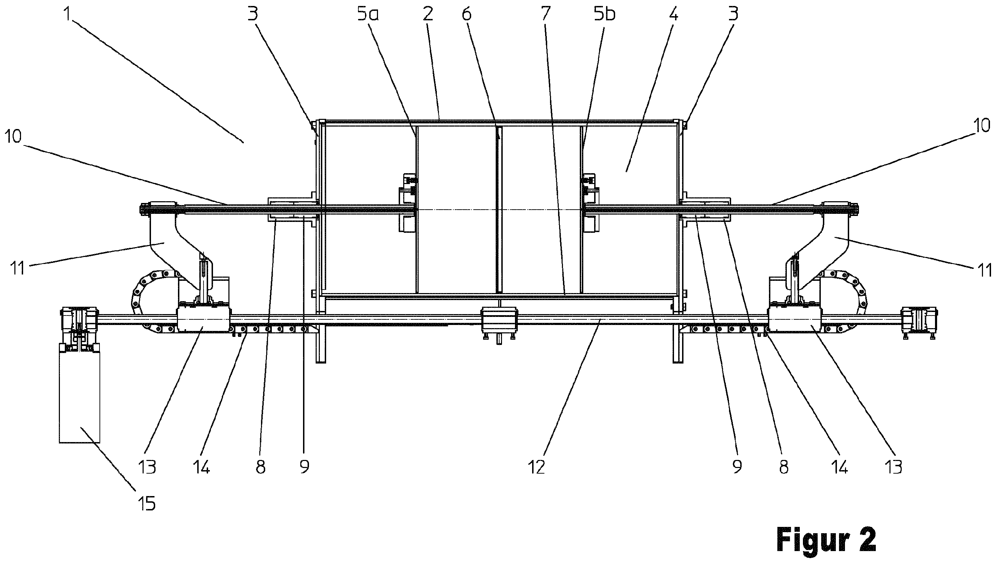

Figur 2 zeigt den Bioresonanzfrequenz-Signalresonator (1) im vertikalen medialen Längsschnitt mit einem mittleren Spiegelabstand von 21,54 cm, dem die Bioresonanzfrequenz des Herzens zugeordnet ist (s. Tabelle 1).

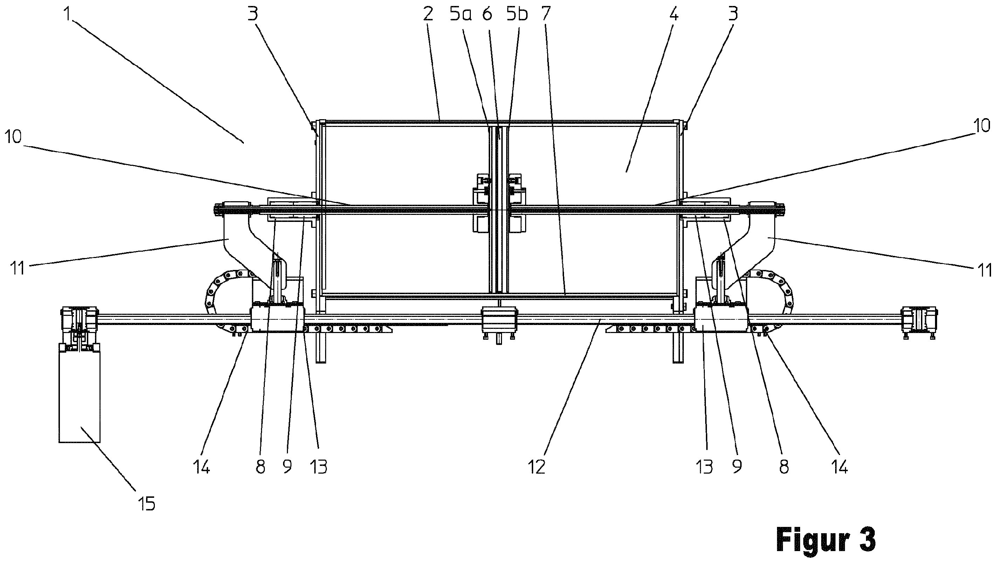

Figur 3 zeigt den Bioresonanzfrequenz-Signalresonator 1 im vertikalen medialen Längsschnitt mit einem geringen Spiegelabstand von beispielsweise 3,10 cm, dem eine Bioresonanzfrequenz des Dünndarms zugeordnet ist (s. Tabelle 1). Dies wurde deshalb so gewählt, damit der Mittenabgriff (6) nicht die Spiegel (5a, 5b) teilweise in der Figur verdeckt.

Es ist aber auch jeder andere, beliebige Abstand der Spiegel (5a, 5b) anfahrbar, wobei bevorzugt die Angaben aus der obigen Tabelle angewendet werden.

Bezugszeichenliste

| | |
|---|---|
| 1 | Bioresonanzfrequenz-Signalresonator |
| 2 | Zylindermantel |
| 3 | Abschlussbleche |
| 4 | Hohlraum |
| 5a, 5b | Spiegel |
| 6 | Mittenabgriff |
| 7 | Abschirmung |
| 8 | Führungsflansche |
| 9 | Bohrung |
| 10 | Hohlwellen |
| 11 | Träger |
| 12 | Zahnriemenachse |
| 13 | Schlitten |
| 14 | Leistungsketten |
| 15 | Elektromotor |

**Patentansprüche**

1. Bioresonanzfrequenz-Signalresonator (1) mit einem zylindrischen Resonanzraum, der zur Einkopplung einer Frequenz von 0,55 mHz, 0,95 mHz, 3,50 mHz, 25,00 mHz, 55,00 mHz, 3,00 MHz, 3,90 MHz, 12,50 MHz, 15,00 MHz, 22,00 MHz, 24,00 MHz, 36,00 MHz, 98,00 MHZ, 148,00 MHz, 240,00 MHz, 270,00 MHz, 384,00 MHz, oder 730,00 MHz in den Resonanzraum eingerichtet ist, **umfassend:**

   a) einen aus einem magnetisch absorbierenden Metall bestehenden Zylindermantel (2) mit kreisförmigen Abschlussblechen (3), die einen zylindrischen Hohlraum (4) einschließen,

   b) zwei innerhalb des Zylindermantels (2) auf dessen Mittellinie axial gegenüberliegende, mittels eines Antriebsgetriebes (10, 11, 12, 15) synchron über gleiche Wegstrecken verschieb- und justierbare, als Kreisscheiben ausgebildete Spiegel (5a, 5b),

   c) eine den Hohlraum (4) abdeckende elektromagnetische Abschirmung (7), die sich innen auf dem Zylindermantel (2) und den Abschlussblechen (3) befindet,

   d) wobei der Bioresonanzfrequenz-Signalresonator (1) weiterhin einen in der axialen Mitte, innerhalb des Zylindermantels (2), ortsfest angeordneten Mittenabgriff (6) zum Auskoppeln von elektromagnetischen Signalen umfasst, wobei der Bioresonanzfrequenz-Signalresonator (1) **dadurch gekennzeichnet ist, dass**

   e) der Mittenabgriff (6) ringförmig ist,

f) die Spiegel (5a, 5b) parabolisch und synchron über gleiche Wegstrecken, aber in entgegengesetzte Richtungen verschieb- und justierbar sind, und

g) die Spiegel (5a, 5b) und der Mittenabgriff (6) jeweils über Leitungen zur Signalübertragung mit externen Geräten verfügen,

h) wobei die zu den Spiegeln (5a, 5b) verlaufenden Leitungen eingerichtet sind, das einzukoppelnde Bioresonanzfrequenz-Signal zu übertragen, und die Leitung vom Mittenabgriff (6) eingerichtet ist, das auszukoppelnde Signal zu übertragen.

2. Bioresonanzfrequenz-Signalresonator (1) nach Patentanspruch 1, wobei das Antriebsgetriebe zwei Hohlwellen (10), zwei Träger (11), eine Zahnriemenachse (12) und einen Elektromotor (15) umfasst.

3. Bioresonanzfrequenz-Signalresonator (1) nach Patentanspruch 1 oder 2, wobei der Bioresonanzfrequenz-Signalresonator (1) einen Grundrahmen aufweist.

4. Bioresonanzfrequenz-Signalresonator (1) nach einem der vorstehenden Patentansprüche, wobei die Leitungen zur Signalübertragung mit den externen Geräten Koaxialleitungen sind, die jeweils mit einer Koaxialsteckerbuchse im Grundrahmen verbunden sind.

5. Bioresonanzfrequenz-Signalresonator (1) nach Patentanspruch 4, wobei der Mittenabgriff (2) aus einem Kupferring oder einer kupfernen Spule besteht und die zugehörige Koaxialleitung angelötet ist.

6. Bioresonanzfrequenz-Signalresonator (1) nach einem der Patentansprüche 2 bis 5, wobei die Spiegel (5a, 5b) von den Hohlwellen (10) getragen werden, die mit den Trägern (11) verbunden sind, wobei diese ihrerseits auf Schlitten (13) der Zahnriemenachse (12) befestigt sind.

7. Bioresonanzfrequenz-Signalresonator (1) nach einem der vorstehenden Patentansprüche, wobei die Spiegel (5a, 5b) aus einem entsprechend den Anforderungen des Standards UL94V-0 schwer entflammbaren Verbundwerkstoff aus Epoxidharz und Glasfasergewebe bestehen, der beidseitig mit Kupfer beschichtet ist, und wobei die einander zugewandten Seiten der Spiegel (5a, 5b) mit Gold bedampft sind.

8. Bioresonanzfrequenz-Signalresonator (1) nach Patentanspruch 6, wobei der eine Schlitten (13) der Zahnriemenachse (12) mit dem Obertrum des Zahnriemens und der andere Schlitten (13) der Zahnriemenachse (12) mit dem Untertrum verbunden ist.

9. Bioresonanzfrequenz-Signalresonator (1) nach einem der Patentansprüche 4 bis 8, wobei Leistungsketten (14) einerseits an den Trägern (11) und andererseits am Grundrahmen befestigt sind und

- die an den Spiegeln (5a, 5b) angelöteten Koaxialleitungen durch die Hohlwellen (10), die Leistungsketten (14) zu Koaxialsteckerbuchsen im Grundrahmen geführt sind.

10. Bioresonanzfrequenz-Signalresonator (1) nach einem der Patentansprüche 3 bis 9, wobei am oder im Grundrahmen eine Bedienungseinheit mit Tastatur, Speicher und Display oder Touchscreen zur Steuerung des Elektromotors (15) des Bioresonanzfrequenz-Signalresonators (1) vorhanden ist, und wobei das Display oder der Touchscreen die eingekoppelten und die ausgekoppelten Signale anzeigen kann.

**Claims**

1. A bioresonance frequency signal resonator (1) with a cylindrical resonance chamber designed for coupling in a frequency of 0.55 mHz, 0.95 mHz, 3.50 mHz, 25.00 mHz, 55.00 mHz, 3.00 MHz, 3.90 MHz, 12.50 MHz, 15.00 MHz, 22.00 MHz, 24.00 MHz, 36.00 MHz, 98.00 MHz, 148.00 MHz, 240.00 MHz, 270.00 MHz, 384.00 MHz, or 730.00 MHz in the resonance chamber, **comprising:**

a) a cylinder jacket (2) made of a magnetically absorbent metal with circular end plates (3) enclosing a cylindrical cavity (4),

b) two mirrors (5a, 5b) arranged within the cylinder jacket (2) on its centre line, axially opposite each other, synchronously movable and adjustable over equal distances via a drive gear (10, 11, 12, 15), and designed as circular discs,

c) an electromagnetic shield (7) covering the cavity (4), located on the inside of the cylinder jacket (2) and the end plates (3),

d) wherein the bioresonance frequency signal resonator (1) further comprises a centre tap (6) arranged in the axial centre within the cylinder jacket (2), for decoupling electromagnetic signals, wherein the bioresonance frequency signal resonator (1) is **characterised in that**

e) the centre tap (6) is annular,

f) the mirrors (5a, 5b) are parabolic and can be moved and adjusted synchronously over equal distances but in opposite directions, and

g) the mirrors (5a, 5b) and the centre tap (6) each have lines to transmit signals to external devices,

h) wherein the lines extending to the mirrors (5a, 5b) are configured to transmit the bioresonance frequency signal to be coupled, and the line from the centre tap (6) is configured to transmit the signal to be decoupled.

2. A bioresonance frequency signal resonator (1) according to patent claim 1, wherein
the drive gear comprises two hollow shafts (10), two carriers (11), a toothed belt axis (12) and an electric motor (15).

3. A bioresonance frequency signal resonator (1) according to patent claim 1 or 2, wherein
the bioresonance frequency signal resonator (1) has a base frame.

4. A bioresonance frequency signal resonator (1) according to one of the preceding patent claims, wherein
the lines to transmit signals to external devices are coaxial lines, each of which is connected to a coaxial connector socket in the base frame.

5. A bioresonance frequency signal resonator (1) according to patent claim 4, wherein
the centre tap (2) consists of a copper ring or a copper coil and the associated coaxial cable is soldered on.

6. A bioresonance frequency signal resonator (1) according to one of patent claims 2 to 5, wherein
the mirrors (5a, 5b) are supported by hollow shafts (10) connected to the carriers (11), which in turn are mounted on sliders (13) of the toothed belt axis (12).

7. A bioresonance frequency signal resonator (1) according to one of the preceding patent claims, wherein

the mirrors (5a, 5b) are made of a composite material consisting of epoxy resin and glass fibre fabric that is flame-retardant in accordance with the requirements of standard UL94V-0 and coated with copper on both sides, and wherein

the sides of the mirrors (5a, 5b) facing each other are vapour-coated with gold.

8. A bioresonance frequency signal resonator (1) according to patent claim 6, wherein
one slider (13) of the toothed belt axis (12) is connected to the toothed belt's upper run and the other slider (13) of the toothed belt axis (12) is connected to the lower run.

9. A bioresonance frequency signal resonator (1) according to one of patent claims 4 to 8, wherein

Service chains (14) are on one side attached to the carriers (11) and on the other side to the base frame and the coaxial cables soldered to the mirrors (5a, 5b) are routed through the hollow shafts (10) and the service chains (14) to coaxial connector sockets in the base frame.

10. A bioresonance frequency signal resonator (1) according to one of patent claims 3 to 9, wherein
an operating unit with a keyboard, memory and display or touchscreen for controlling the electric motor (15) of the bioresonance frequency signal resonator (1) is provided on or in the base frame, and wherein the display or touchscreen can show the coupled and decoupled signals.

**Revendications**

1. Résonateur de signal de fréquence de biorésonance (1) avec une chambre de résonance cylindrique, conçu pour coupler en entrée une fréquence de 0,55 mHz, 0,95 mHz, 3,50 mHz, 25,00 mHz, 55,00 mHz, 3,00 MHz, 3,90 MHz, 12,50 MHz, 15,00 MHz, 22,00 MHz, 24,00 MHz, 36,00 MHz, 98,00 MHz, 148,00 MHz, 240,00 MHz, 270,00 MHz, 384,00 MHz ou 730,00 MHz dans la chambre de résonance, comprenant :

a) une enveloppe de cylindre (2) constituée d'un métal magnétiquement absorbant et munie de plaques d'extrémité (3) circulaires entourant une cavité cylindrique (4),

b) deux miroirs (5a, 5b) réalisés sous la forme de disques circulaires et pouvant être déplacés et réglés de manière synchrone sur des distances égales au moyen d'un mécanisme d'entraînement (10, 11, 12, 15) et se faisant face à l'intérieur de l'enveloppe de cylindre (2) de manière axiale sur l'axe médian de ladite enveloppe,

c) un blindage électromagnétique (7) recouvrant la cavité (4) et située à l'intérieur de l'enveloppe de cylindre (2) et des plaques d'extrémité (3),

d) dans lequel le résonateur de signal de fréquence de biorésonance (1) comprend en outre une prise centrale (6) agencée dans une position fixe sur le centre axial à l'intérieur de l'enveloppe de cylindre (2) et permettant de coupler en sortie des signaux électromagnétiques, dans lequel le résonateur de signal de fréquence de biorésonance (1) est **caractérisé en ce que**

e) la prise centrale (6) est en forme d'anneau,

f) les miroirs (5a, 5b) sont paraboliques et peuvent être déplacés et réglés de manière synchrone sur des distances égales, mais dans des directions opposées, et

g) les miroirs (5a, 5b) et la prise centrale (6) disposent respectivement de lignes permettant de transmettre un signal vers des appareils externes,

h) dans lequel les lignes menant aux miroirs (5a, 5b) sont conçues pour transmettre le signal de fréquence de biorésonance à coupler en entrée, et la ligne provenant de la prise centrale (6) est configurée pour transmettre le signal à coupler en sortie.

2. Résonateur de signal de fréquence de biorésonance (1) selon la revendication 1, dans lequel le mécanisme d'entraînement comprend deux arbres creux (10), deux supports (11), un axe de courroie crantée (12) et un moteur électrique (15).

3. Résonateur de signal de fréquence de biorésonance (1) selon la revendication 1 ou 2, dans lequel le résonateur de signal de fréquence de biorésonance (1) présente un châssis de base.

4. Résonateur de signal de fréquence de biorésonance (1) selon l'une quelconque des revendications précédentes, dans lequel les lignes permettant de transmettre un signal vers les appareils externes sont des câbles coaxiaux qui sont connecté respectivement à une fiche coaxiale dans le châssis de base.

5. Résonateur de signal de fréquence de biorésonance (1) selon la revendication 4, dans lequel la prise centrale (2) est constituée d'un anneau en cuivre ou d'une bobine en cuivre et le câble coaxial associé est soudé dessus.

6. Résonateur de signal de fréquence de biorésonance (1) selon l'une quelconque des revendications 2 à 5, dans lequel les miroirs (5a, 5b) sont portés par les arbres creux (10) qui sont reliés aux supports (11), ceux-ci étant à leur tour sont fixés sur des chariots (13) de l'axe de courroie crantée (12).

7. Résonateur de signal de fréquence de biorésonance (1) selon l'une quelconque des revendications précédentes, dans lequel

les miroirs (5a, 5b) sont constitués d'un matériau composite ignifuge composé de résine époxy et de tissu de fibre de verre et recouvert de cuivre sur les deux faces, conformément aux exigences de la norme UL94V-0, et dans lequel

de l'or est vaporisé sur les côtés mutuellement opposés des miroirs (5a, 5b).

8. Résonateur de signal de fréquence de biorésonance (1) selon la revendication 6, dans lequel un chariot (13) de l'axe de courroie crantée (12) est relié au brin supérieur de la courroie crantée et l'autre chariot (13) de l'axe de courroie crantée (12) est relié au brin inférieur.

9. Résonateur de signal de fréquence de biorésonance (1) selon l'une quelconque des revendications 4 à 8, dans lequel

des chaînes porte-câbles (14) sont fixées d'une part aux supports (11) et d'autre part au châssis de base et les câbles coaxiaux soudés aux miroirs (5a, 5b) sont guidés à travers les arbres creux (10) et les chaînes porte-câbles (14) vers des fiches coaxiales dans le châssis de base.

**10.** Résonateur de signal de fréquence de biorésonance (1) selon l'une quelconque des revendications 3 à 9, dans lequel sur ou dans le châssis de base se trouve une unité de commande avec un clavier, une mémoire et un écran ou un écran tactile afin de commander le moteur électrique (15) du résonateur de signal de fréquence de biorésonance (1), et dans lequel l'écran ou l'écran tactile peut afficher les signaux couplés en entrée et en sortie.

**Figur 1**

**Figur 2**

Figur 3

**IN DER BESCHREIBUNG AUFGEFÜHRTE DOKUMENTE**

**In der Beschreibung aufgeführte Patentdokumente**

- DE 2757716 A1
- DE 4020881 A1
- DE 202013100991 U1
- DE 102015006368 A1
- US 3161840 A
- US 2460090 A
- US 2281550 A